Europäisches Patentamt

European Patent Office

Office européen des brevets

(11) Veröffentlichungsnummer: **0 043 158**
**B1**

(12) **EUROPÄISCHE PATENTSCHRIFT**

(45) Veröffentlichungstag der Patentschrift:
11.09.85

(21) Anmeldenummer: 81200695.5

(22) Anmeldetag: 19.06.81

(51) Int. Cl.⁴: **G 01 S 7/52, G 01 S 15/89,**
**G 01 N 29/04, A 61 B 10/00**

(54) Ultraschall-Untersuchungsanordnung.

(30) Priorität: 02.07.80 DE 3024995

(43) Veröffentlichungstag der Anmeldung:
06.01.82 Patentblatt 82/1

(45) Bekanntmachung des Hinweises auf die Patenterteilung:
11.09.85 Patentblatt 85/37

(84) Benannte Vertragsstaaten:
DE FR GB SE

(56) Entgegenhaltungen:
GB - A - 2 023 830
US - A - 3 852 745
US - A - 4 063 549

IEEE 1979 ULTRASONICS SYMPOSIUM, Proceedings, New Orleans, Louislana 26-28 September 1979 NEW YORK (US) Catalog Nr. 79CH1482-9 M. O'DONNELL u.a.: "Broadband Integrated Backscatter: An Approach to Spatially Localized Tissue Characterization In Vivo" Seiten 175-178
PHYSICS IN MEDICINE & BIOLOGY, Band 22, No. 2, März 1977 LONDON (GB) J.C. GORE u.a.: "Ultrasonic Backscattering from Human Tissue: A Realistic Model" ACOUSTICAL IMAGING, Band 8, 1978, Herausgeber: A.F. NETHERELL PLENUM PRESS NEW YORK (US) S.A. JOHNSON u.a.: "Algebraic and Analytic Inversion of Acoustic Data from Partlally or Fully Enclosing Apertures", Seiten 577-598
ACOUSTICAL IMAGING: Band 9, 1979, Herausgeber:

(73) Patentinhaber: Philips Patentverwaltung GmbH, Billstrasse 80, D-2000 Hamburg 28 (DE)

(84) Benannte Vertragsstaaten: DE

(73) Patentinhaber: N.V. Philips' Gloeilampenfabrieken, Groenewoudseweg 1, NL-5621 BA Eindhoven (NL)

(84) Benannte Vertragsstaaten: FR GB SE

(72) Erfinder: Schomberg, Hermann, Dr. Dipl.-Math., König-Heinrich-Weg 127 d, D-2000 Hamburg 61 (DE)

(74) Vertreter: Hartmann, Heinrich, Dipl.-Ing. et al, Philips Patentverwaltung GmbH Billstrasse 80 Postfach 10 51 49, D-2000 Hamburg 28 (DE)

(56) Entgegenhaltungen: (Fortsetzung)
K.Y. WANG, PLENUM PRESS NEW YORK (US) J. BALL u.a.: "Explicit Inversion of the Helmholtz Eqiation for Ultra-Sound Insonification and Spherical Detection", Seiten 451-461
IEEE TRANSACTIONS ON BIOMEDICAL ENGINEERING, Band BME-27, no. 9 September 1980 NEW YORK (US) R.A. ALTES u.a.: "A Unified Method of Broadband Echo Characterization for Diagnostic Ultrasound", Selten 500-508
IEEE TRANSACTIONS ON BIOMEDICAL ENGINEERING, Band BME-28, No. 2, Februar 1981 NEW YORK (US) S.J. MORTON u.a.: "Ultrasonic Reflectivity Imaging in Three Dimensions: Exact Inverse Scattering Solutions for Plane, Cylindrical and Spherical Apertures"

## Beschreibung

Die Erfindung betrifft eine Anordnung nach dem Oberbegriff des Hauptanspruches.

Eine solche Anordnung ist im wesentlichen aus der US-PS 38 81 466 [1] bekannt. Die bekannte Anordnung besitzt eine Zeile von Schallwandlerelementen, mit denen Schallwellen erzeugt und empfangen werden können. Dabei werden mittels einer Steuerschaltung nacheinander jeweils unterschiedliche Gruppen benachbarter Schallwandlerelemente kurzzeitig zur Erzeugung eines Schallstrahles angesteuert und die jeweils ankommenden Schallechoimpulse gemessen, so daß der Körper auf diese Weise abgetastet wird. Mittels einer elektronischen Einheit ist aus diesen Schallechoimpulsen dann ein Querschnittsbild des Körpers rekonstruierbar, indem z. B. die gemessenen Schallechoimpulse nach einer analogen Vorverarbeitung in Abhängigkeit vom Ort des jeweiligen Empfängers und entsprechend ihrer Laufzeit als ebene Helligkeitsverteilung dargestellt werden, wobei sich die Helligkeit nach der Amplitude der Schallechoimpulse richtet. Anordnungen dieser Art werden üblicherweise als B-Scan-Anordnungen bezeichnet. Sie liefern jedoch nur annähernd gültige Aussagen über die Lage akustischer Grenzflächen im beschallten Körperteil, d. h., es werden nur solche Grenzflächen registriert, die auf den Sender zurückstrahlen, also wenigstens annähernd senkrecht zur Strahlrichtung stehen. Ferner kann nicht die Größe und das Vorzeichen eines Impedanzsprunges an einer Grenzfläche ermittelt werden. Die Lagebestimmung einer Grenzfläche ist ebenfalls ungenau, weil bei der Rekonstruktion eine konstante Schallgeschwindigkeit zugrundegelegt wird, was nur näherungsweise zutrifft. Die Ungenauigkeit wird weiter dadurch erhöht, daß sich zunächst parallele Schallstrahlen auch kreuzen können, wodurch ›links und rechts‹ vertauscht wird. Außerdem werden Mehrfachechos mit verarbeitet, so daß unter Umständen in Wirklichkeit nicht existierende Grenzflächen dargestellt werden.

Eine Anordnung nach dem Oberbegriff des Hauptanspruches ist auch aus der GB-A- 2 023 830 bekannt. Da dabei die Transmission der Ultraschallenergie untersucht wird, sind wenigstens zwei Schallwandleranordnungen erforderlich, die das Objekt anschließen und von denen die eine dem Senden und die andere dem Empfang eines Ultraschallsignals dient.

Aus der US-A- 4 063 549 ist eine Ultraschalluntersuchungsanordnung bekannt mit einem Schallwandler, der eine impulsförmige Schallwelle aussendet, die mittels eines elliptischen akustischen Reflektors auf ein Objekt fokussiert wird, die reflektierte Ultraschallenergie wird von dem gleichen Wandler gemessen, das Meßsignal wird abgetastet und digitalisiert und einer Fourier-Transformation unterworfen. Durch Division der einzelnen Werte durch die Fourier-Transformation des Sendesignals des Ultraschallwandlers wird die Übertragungsfunktion des Objektpunktes erhalten, auf den die Ultraschallstrahlung fokussiert ist. Durch Rücktransformation läßt sich daraus der zeitliche Verlauf der akustischen Impedanz dieses Objektpunktes ermitteln und auf einer Wiedergabeeinheit darstellen. Wiederholt man dies für verschiedene Wandlerpositionen, dann läßt sich bei entsprechender Steuerung der Wiedergabeanordnung eine zweidimensionale Darstellung der akustischen Impedanz des im Fokus befindlichen Objektteiles erreichen.

Weiterhin ist aus der US-A- 3 852 745 eine Ultraschall-Untersuchungsanordnung bekannt, bei der ein Untersuchungsobjekt von einem ersten Wandler angestrahlt wird, der kontinuierlich von einem sinusförmigen Signal erregt wird. Das von dem Objekt reflektierte Signal wird von einer Wandlermatrix in elektrische Signale umgesetzt, die nach Mischung mit einem weiteren sinusförmigen Signal abgetastet werden, wobei für jeden Wandler dieser Wandlermatrix die Amplitude und die Phase der empfangenen Ultraschallwelle bestimmt wird. Mit den auf diese Weise erhaltenen Werten der Ultraschallwandler wird eine räumliche Fourier-Transformation durchgeführt, deren Ergebnis auf einem Wiedergabegerät dargestellt wird.

Weiterhin ist aus einem Aufsatz in IEEE 1979 Ultrasonic Symposium, Proceedings, 26. – 28. Sept. 1979 in New Orleans, Seiten 175 bis 178, eine Ultraschall-Untersuchungsanordnung bekannt, bei der ein Objektbereich mit einem Ultraschallimpuls bestrahlt wird, dessen Echo von dem gleichen Wandler aufgenommen, in ein elektrisches Signal umgesetzt, digitalisiert und fouriertransformiert wird. Das auf diese Weise erhaltene Frequenzspektrum des Echoimpulses wird mittels einer Wiedergabeeinheit dargestellt.

Aufgabe der vorliegenden Erfindung ist es, eine Ultraschall-Untersuchungsanordnung der eingangs genannten Art so auszugestalten, daß damit verbesserte und quantitative Aussagen über die innere Struktur eines Körpers gemacht werden können. Diese Aufgabe wird durch die im Kennzeichen des Anspruches 1 angegebenen Maßnahmen gelöst.

Mittels einer derartigen Anordnung ist es möglich, die innere Struktur eines Körpers relativ genau zu rekonstruieren, da u. a. quantitative Ergebnisse hinsichtlich des Realteils $n(\vec{r})$ und des Imaginärteils $n(\vec{r}) \cdot k(\vec{r})$ des komplexen Brechungsindex $\tilde{n}(\vec{r})$ im beschallten Körperbereich gewonnen werden. Fehler aufgrund von Mehrfachechos oder Kreuzungen von Schallwellen können größtenteils vermieden werden, da die der Rekonstruktion zugrundeliegende Integralgleichung die Schallausbreitung vollständig beschreibt.

Die Anordnung kann vorteilhaft bei der Untersuchung von Körpern eingesetzt werden, in denen sich keine zu großen Impedanzsprünge befinden. Insbesondere eignet sich die Anordnung für Mammauntersuchungen zur Brustkrebserkennung und -diagnose, zur Untersuchung des Abdomens und in manchen Fällen auch zur Materialprüfung.

Die Zeichnungen stellen Ausführungsbeispiele der Erfindung dar. Es zeigt

Fig. 1 eine Skizze zur Erläuterung der Schallausbreitung in einem zu untersuchenden Körper,

Fig. 2 ein Impuls-Zeitdiagramm zur Darstellung eines Meßzyklus,

Fig. 3 ein Block- und Signaldiagramm zur Erläuterung der Verarbeitung eines Meßsignals entlang eines Meßsignalweges,

Fig. 4 ein Blockdiagramm einer Ultraschall-Untersuchungsanordnung mit einer Zeile von Schallwandlerelementen,

Fig. 5 a—d unterschiedliche Ausführungsformen von Schallwandleranordnungen der Ultraschall-Untersuchungsanordnung.

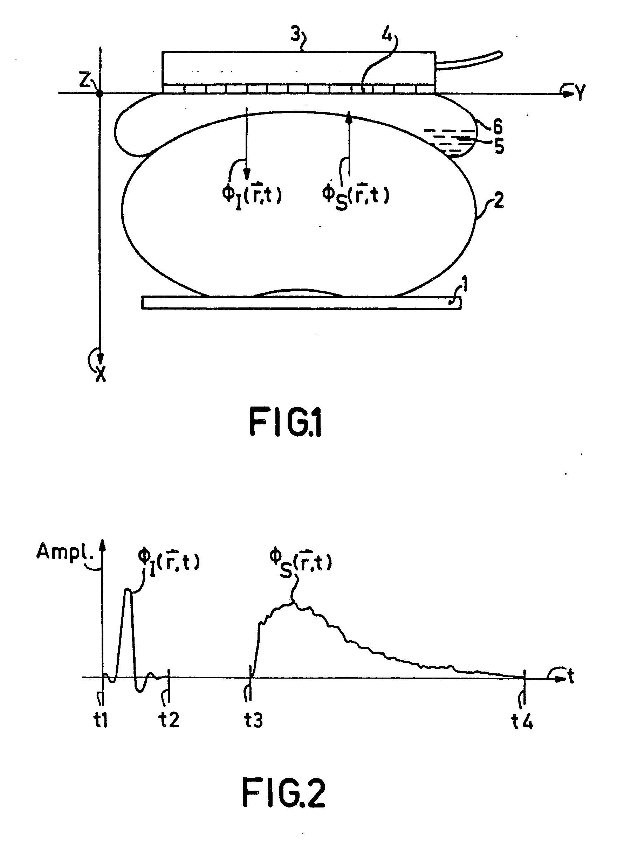

In der Fig. 1 ist ein Querschnitt durch einen auf einem Aufnahmetisch 1 liegenden Körper 2 dargestellt, auf dem eine Schallwandleranordnung 3 aufliegt, deren Schallwandlerelemente 4 in Form einer zweidimensionalen Matrix angeordnet sind und sowohl Schallwellen aussenden als auch empfangen können. Die Schallwandlerelemente können also sowohl als Schallsendeelemente als auch als Schallempfängerelemente betrieben werden. Die Schallwandleranordnung 3 ist über ein Schallkoppelmedium 5, z. B. Wasser, akustisch an den Körper 2 gekoppelt, das sich innerhalb eines von einer mit der Schallwandleranordnung 3 flüssigkeitsdicht verbundenen elastischen Folie 6 begrenzten Raumes befindet. In der Ebene der Schallwandlerelemente 4 liegt die Y, Z-Ebene eines dreidimensionalen kartesischen Koordinatensystems XYZ, wobei die Y-Achse in Richtung der Matrixzeilen und die X-Achse senkrecht zur Matrixebene in Richtung des Körpers 2 verläuft.

Anhand der Figuren 1 und 2 sollen im folgenden die wichtigsten Überlegungen erläutert werden, die dem Aufbau der erfindungsgemäßen Ultraschall-Untersuchungsanordnung zugrundeliegen. Dabei darf es nicht als Einschränkung verstanden werden, daß die Schallwandleranordnung hier zweidimensional und jeweils zum Senden und Empfangen von in gleicher Richtung verlaufenden Schallwellen ausgebildet ist. Ebenso kann die Schallwandleranordnung in anderer geeigneter Weise, z. B. zeilenförmig, oder derart ausgebildet sein, daß sie zur Aussendung und zum Empfang von in jeweils unterschiedlichen Richtungen verlaufenden Schallwellen geeignet ist, was weiter unten noch genauer erläutert wird.

Die Ermittlung der inneren Struktur des Körpers 2 mittels der Ultraschall-Untersuchungsanordnung erfolgt in der Weise, daß mit Hilfe der Schallwandleranordnung 3 eine nahezu ebene Schallwelle $\Phi_i(\vec{r}, t)$ in den Körper 2 eingestrahlt wird, indem z. B. alle Schallwandlerelemente 4 gleichzeitig angeregt werden. Die eingestrahlte Schallwelle $\Phi_i(\vec{r}, t)$ muß dabei möglichst impulsförmig sein, damit ihr Frequenzspektrum möglichst breitbandig ist. Beispielsweise könnte die eingestrahlte Schallwelle $\Phi_i(\vec{r}, t)$ bei festem $\vec{r}$ als Funktion von t wenigstens annähernd eine Deltafunktion sein.

Dies ist erforderlich, damit die Frequenz $\omega$ als unabhängige Variable bei der Bestimmung z. B. der dreidimensionalen inneren Körperstruktur mittels einer zweidimensionalen Schallwandleranordnung verwendet werden kann. Beispielsweise könnte die Mittenfrequenz zwischen 0,5 und 1,5 MHz liegen.

In Fig. 2 ist eine solche in den Körper 2 eingestrahlte Schallwelle $\Phi_i(\vec{r}, t)$ bei festem $\vec{r}$ als Funktion der Zeit t zwischen den Zeiten t1 und t2 dargestellt. Die eingestrahlte Schallwelle läßt sich in der Form

$$\Phi_i(\vec{r}, t) = \int\limits_{-\infty}^{\infty} A(\omega)\, e^{i\left(\frac{\omega}{Co}x - \omega t\right)} d\omega \tag{7}$$

schreiben, wobei $A(\omega)$ die frequenzabhängige Amplitude bzw. das Frequenzspektrum der eingestrahlten Schallwelle $\Phi_i(\vec{r}, t)$ am Ort der Schallwandlerelemente 4 (x = 0), $\omega$ die Frequenz der einzelnen Wellenanteile, t die Zeit, x der Ortsabstand in Richtung der Koordinatenachse X und Co die Schallgeschwindigkeit der Schallwellen, z. B. in Wasser bei Zimmertemperatur, ist. Die Funktion $A(\omega)$ ist dabei bekannt bzw. kann durch Messung von $\Phi_i(\vec{r}, t)$ und Fouriertransformation ermittelt werden. Sie berücksichtigt sowohl die reinen Übertragungseigenschaften der einzelnen Schallwandlerelemente 4 beim Aussenden der Schallwellen als auch die Form der die Schallwandlerelemente 4 anregenden elektrischen Eingangssignale. Nachdem die eingestrahlte Schallwelle $\Phi_i(\vec{r}, t)$ abgeklungen ist, können z. B. alle Schallwandlerelemente 4 zum Zeitpunkt t3 $\geq$ t2 auf Empfang geschaltet werden, so daß mit ihrer Hilfe die an den Körperstrukturen gestreuten Schallwellen $\Phi_s(\vec{r}, t)$, die ebenfalls in Fig. 2 dargestellt sind, gemessen werden können, und zwar für x = 0, d. h. in der Wandlerebene. Die Schallwelle $\Phi_s(\vec{r}, t)$ ist durch die Gleichung

$$\Phi(\vec{r}, t) = \Phi_i(\vec{r}, t) + \Phi_s(\vec{r}, t) \tag{8}$$

definiert, wobei $\Phi(\vec{r}, t)$ die gesamte im Körper 2 verlaufende Schallwelle ist; sie stellt die physikalische Observable dar. Aufgrund der zeitlichen Verschiebung der Schallwellenerzeugung und -messung ist also bis zum Zeitpunkt t2 die Observable $\Phi(\vec{r}, t)$ gleich $\Phi_i(\vec{r}, t)$, wobei $\Phi_i(\vec{r}, t)$ durch Gl. (7) definiert ist. Die gestreute und durch Gl. (8) definierte Schallwelle $\Phi_s(\vec{r}, t) = \Phi(\vec{r}, t) - \Phi_i(\vec{r}, t)$ stimmt aber in der Meßphase t $\geq$ t3 (vergl. Fig. 2), in der $\Phi_i(\vec{r}, t)$ abgeklungen ist, mit der Observablen überein, so daß dann $\Phi_s(\vec{r}, t)$ gemessen wird. Ein Meßzyklus, der aus dem Aussenden der impulsförmigen Schallwelle $\Phi_i(\vec{r}, t)$ und dem Empfangen der gestreuten Schallwelle $\Phi_s(\vec{r}, t)$ besteht, ist dann, nachdem die gestreute Schallwelle $\Phi_s(\vec{r}, t)$ abgeklungen ist, zum Zeitpunkt t4 beendet. Für den Fall, daß der Weg der Schallwellen zwischen der Schallwandleranordnung 3 und dem Körper 4 nur sehr kurz ist, z. B. bei Verwendung eines Kontaktgels statt des Wasserreservoirs, kann die gestreute Schallwelle $\Phi_s(\vec{r}, t)$ noch vor Beendigung der Schallaussendung die Schallwandleranordnung 3 erreichen. In diesem Fall könnten dann die der Schallwandleranordnung 3 am nächsten liegenden Bereiche des Körpers 2 nicht rekonstruiert werden.

Die gestreute Schallwelle kann in der Form

$$\Phi_s(\vec{r}, t) = \int_{-\infty}^{\infty} A(\omega) \; \Psi_s(\vec{r}, \omega) \; e^{-i\omega t} d\omega \tag{9}$$

geschrieben werden, wobei $\Psi_s(\vec{r}, \omega)$ das Frequenzspektrum der gestreuten Schallwelle darstellt. $\Psi_s(\vec{r}, \omega) \, e^{-i\omega t}$ ist dabei die Welle, die gestreut worden wäre, wenn

$$\Psi_i(\vec{r}, \omega) \; e^{-i\omega t} = c^{i\left(\frac{\omega}{C_0} x - \omega t\right)}$$

eingestrahlt worden wäre. Das mit Hilfe der Schallwandlerelemente 4 jeweils erzeugte elektrische Meßsignal läßt sich dann in der Form

$$\tilde{\Phi}_s(\vec{r}_{ij}, t) = \int_{-\infty}^{\infty} A(\omega) \; B(\omega) \; \Psi_s(\vec{r}_{ij}, \omega) \; e^{-i\omega t} d\omega \tag{10}$$

schreiben, wobei $B(\omega)$ als frequenzabhängige Übertragungsfunktion bezeichnet wird und die Empfangseigenschaften der Schallwandlerelemente beschreibt. $B(\omega)$ ist bekannt oder kann durch geeignete Messung ebenfalls ermittelt werden (vgl. [8], S. 181 ff.).

Die Indices i bzw. j geben dabei das Schallwandlerelement 4 mit den Koordinaten $\vec{r}_{ij} = (0, Y_i, Z_j)$ an, das das Meßsignal $\tilde{\Phi}_s(\vec{r}_{ij}, t)$ übermittelt.

Um das Frequenzspektrum $\Psi_s(\vec{r}_{ij}, \omega)$ aus dem Meßsignal $\tilde{\Phi}_s(\vec{r}_{ij}, t)$ zu ermitteln (Gl. (10)), bedarf es sowohl einer Fouriertransformation des Meßsignals $\tilde{\Phi}_s(\vec{r}_{ij}, t)$ als auch einer Division des fouriertransformierten Meßsignals $\tilde{\Psi}_s(\vec{r}_{ij}, \omega)$ durch die Größen $A(\omega)$ und $B(\omega)$, so daß sich dann das Frequenzspektrum zu

$$\Psi_s(\vec{r}_{ij}, \omega_1) = \frac{\tilde{\Psi}_s(\vec{r}_{ij}, \omega_1)}{A(\omega_1) \; B(\omega_1)} \tag{11}$$

ergibt. In Gleichung (11) indiziert der Index 1 die unterschiedlichen, dem Frequenzspektrum zugehörigen Frequenzen $\omega_1$. Die Division nach Gleichung (11) wird vorzugsweise so ausgeführt, daß das Frequenzspektrum $\tilde{\Psi}_s(\vec{r}_{ij}, \omega_1)$ mit dem Kehrwert eines Korrekturfaktors $C_{ij1}$ multipliziert wird, der z. B. das Produkt $A(\omega_1) \cdot B(\omega_1)$ darstellt. Dies ist vorteilhaft, da der Kehrwert des Korrekturfaktors einmal bestimmt werden kann, und eine Multiplikation schneller als eine Division durchgeführt werden kann. Der Korrekturfaktor $C_{ij1}$ kann aber auch in anderer geeigneter Weise gewählt werden, z. B. zu $C_{ij1} = A(\omega_1) B(\omega_1) + \varepsilon_1$ (Wiener-Filter), um Rauschprobleme optimal behandeln zu können. Sollten die einzelnen Schallwandlerelemente 4 an ihren Orten $(\vec{r}_{ij})$ unterschiedliche Sende- und Empfangseigenschaften besitzen, so wählt man $C_{ij1}$ allgemein zu

$$C_{ij1} = A_{ij}(\omega_1) \; B_{ij}(\omega_1) + \varepsilon_{ij1} \tag{12}$$

wenn zum Aussenden und zum Empfangen von Schallwellen dieselben Schallwandlerelemente verwendet werden.

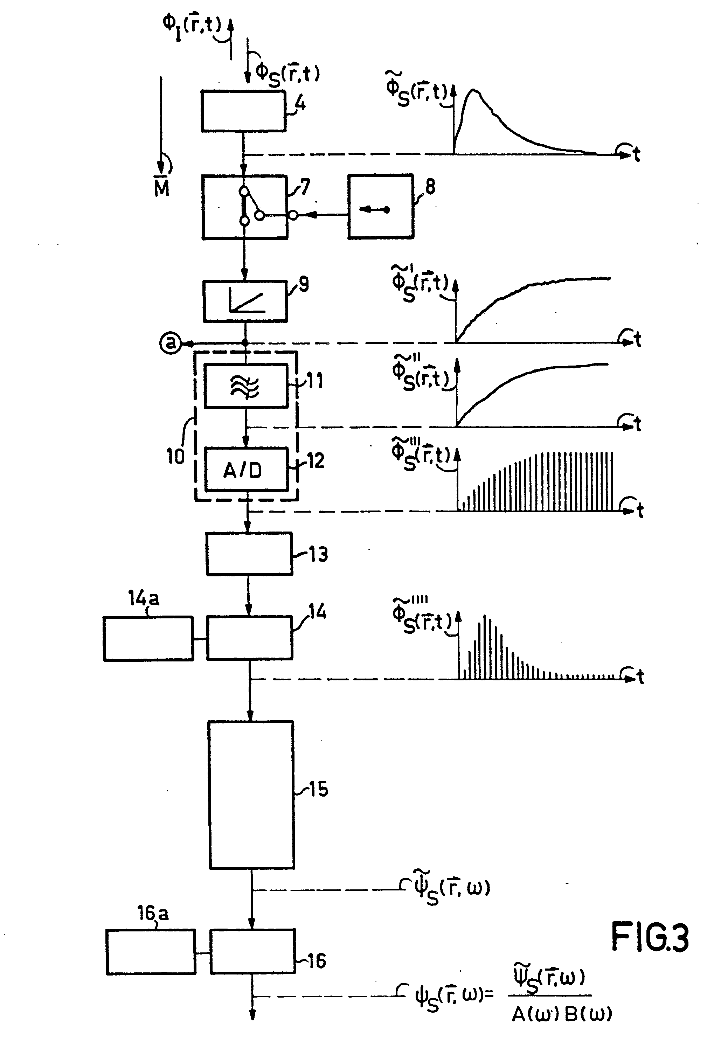

Im folgenden soll auf eine Indizierung (i, j, l) der Übersicht wegen verzichtet werden. Die Erzeugung des Frequenzspektrums $\Psi_s(\vec{r}, \omega)$ aus dem Meßsignal $\tilde{\Phi}_s(\vec{r}, t)$ ist in Fig. 3 beschrieben und wird später genauer erläutert.

Ist das Frequenzspektrum $\Psi_s(\vec{r}, \omega)$ an den Stellen $\vec{r}_{ij}$ und für jeweils unterschiedliche Frequenzen $\omega_1$ bekannt, so läßt sich die innere Körperstruktur hieraus ermitteln.

Ziel ist es, die Integralgleichung (1) für das Potential $V(\vec{r}, \omega)$ zu lösen, d. h. Funktionen f und g zu finden (Gl. (4) und (5)), so daß Gleichung (3) die Integralgleichung (1) erfüllt. Ist z. B. die Funktion f ermittelt worden, so läßt sich daraus über die Gleichungen (4) und (6) u. a. der reelle Brechungsindex $n(\vec{r})$ und der Extinktionskoeffizient $k(\vec{r})$ bestimmen.

Die Lösung der Integralgleichung (1) für $\Psi_s$ an der Stelle $x = 0$, also abgekürzt:

$$\Psi_s|_{x=0} = \int_D G \; V(\Psi_i + \Psi_s) \; d^3r \tag{13}$$

kann iterativ erfolgen. Zuerst wird angenommen, daß $\Psi_s$ sehr klein ist im Vergleich zu $\Psi_i$ (Born'sche Näherung). Dann geht Gl. (13) über in

$$\Psi_S\big|_{x=0} = \int_D G\,V\,\Psi_I\,d^3\dot{r} \tag{14}$$

wobei

$$\Psi_I = e^{i\frac{\omega}{c_0}x} \text{ ist} \tag{14'}$$

Eine derartige Gleichung (14) ist mit Hilfe einer Diskretisierung und dem bekannten

$$\Psi_S\big|_{x=0} \quad (\triangleq \Psi_S(\dot{r}, \omega))$$

numerisch lösbar (vergl. hierzu [9]).

Für den Fall, daß $\Psi_S$ nicht sehr klein gegenüber $\Psi_I$ ist, kann die Lösung von Gl. (14) z. B. mit Hilfe der »Born'schen Reihe« iterativ verbessert werden. Sei etwa

$$V^{(1)} = \omega^2 f^{(1)} + g^{(1)} \tag{15}$$

das Ergebnis nach Gleichung (14), dann läßt sich

$$\Psi_S^{(1)} = \int_D G\,V^{(1)}\,\Psi^{(1)}\,d^3\dot{r} \tag{16}$$

mit

$$\Psi^{(1)} = \Psi_I + \Psi_S^{(0)} \tag{17}$$

und

$$\Psi_S^{(0)} = 0 \tag{18}$$

überall ausrechnen und anschließend

$$\Psi_S\big|_{x=0} = \int_D G\,V(\Psi_I + \Psi_S^{(1)})\,d^3\dot{r} \tag{19}$$

nach V auflösen und das Ergebnis $V^{(2)}$ nennen, usw. Gl. (19) wird dabei wie Gl. (16) gelöst. Nach i Schritten wird das Iterationsverfahren abgebrochen. Aus dem Ergebnis

$$V^{(i)} = \omega^2 f^{(i)} + g^{(i)} \tag{20}$$

läßt sich dann $n(\dot{r})$ und $k(\dot{r})$ bestimmen und graphisch darstellen, z. B. auf einem Monitor, Drucker oder dergleichen.

Zur Ermittlung der Frequenzspektren $\Psi_S(\dot{r}, \omega)$ aus den elektrischen Meßsignalen $\tilde{\Phi}_S(\dot{r}, t)$ besitzt die Ultraschall-Untersuchungsanordnung nach der Erfindung die in Fig. 3 dargestellten Meßsignalwege $\overline{M}$ (auf eine Indizierung (i, j) der Ortsvektoren $\dot{r}$ sei hier der Einfachheit wegen verzichtet). Für den Fall, daß die Ermittlung der inneren Struktur des Körpers 2 mittels eines einzigen Meßzyklus vorgenommen werden soll, muß jedes der Schallwandlerelemente 4 an einen eigenen derartigen Meßsignalweg $\overline{M}$ angeschlossen sein. Es ist aber auch ein sog. Abtastbetrieb möglich, bei dem mehrere Meßzyklen durchlaufen und nach jeweils einer ausgesandten ebenen Schallwelle $\Phi_I(\dot{r}, t)$ jeweils unterschiedliche Gruppen von Schallwandlerelemente 4 auf Empfang geschaltet werden. Dies ist vorteilhaft, da so die Zahl der Meßsignalwege $\overline{M}$ und damit der schaltungstechnische Aufwand einer derartigen Ultraschall-Untersuchungsanordnung verringert werden kann.

Ein Meßsignalweg $\overline{M}$, auf dem das Meßsignal $\tilde{\Phi}_S(\dot{r}, t)$ in Echtzeit verarbeitet werden kann, besitzt z. B. ein Schallwandlerelement 4, mit dessen Hilfe sowohl Schallwellen $\Phi_I(\dot{r}, t)$ ausgesendet als auch empfangen werden können ($\Phi_S(\dot{r}, t)$), und das geeignete Übertragungseigenschaften zur Übertragung impulsförmiger Schallwellen mit breitbandigem Frequenzspektrum aufweist. Das Schallwandlerelement 4 ist mit einem Schalter 7 verbunden, der es beim Aussenden von Schallwellen vom Meßstrahlenweg $\overline{M}$ trennt und elektrisch mit einem Sender 8 verbindet, der die notwendigen elektrischen Signale zur impulsförmigen Anregung des Schallwandlerelementes 4 liefert. Für die Weiterverarbeitung des vom Schallwandlerelement 4 gelieferten elektrischen Meßsignals $\tilde{\Phi}_S(\dot{r}, t)$, das der empfangenen Schallwelle $\Phi_S(\dot{r}, t)$ entspricht, wird dagegen das Schallwandlerelement 4 über den Schalter 7 mit einem Verstärker 9 verbunden, der die Meßsignale $\tilde{\Phi}(\dot{r}, t)$ proportional zur Laufzeit der Schallwellen im Körper 4 verstärkt, so daß auf diese Weise Meßsignale $\tilde{\Phi}_S'(\dot{r}, t)$ mit wenigstens annähernd gleicher Signalstärke entstehen (vergl. [2], Kap. 6).

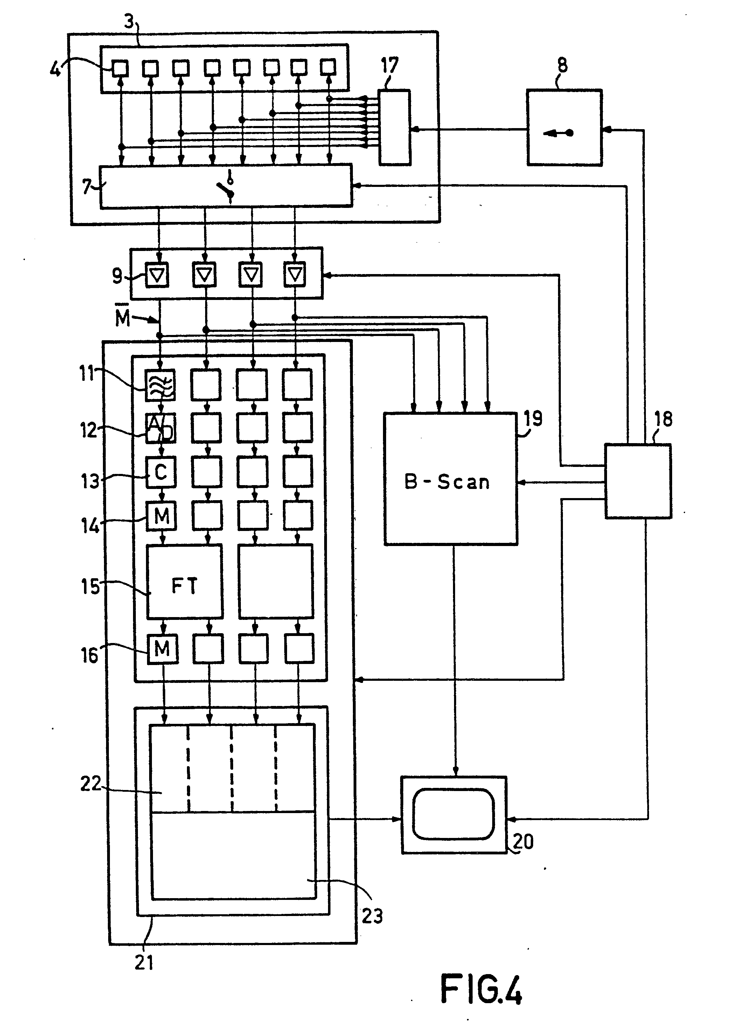

Diese Signale $\tilde{\Phi}_S'(\dot{r}, t)$ können über einen Ausgang a abgegriffen und einer bekannten B-Scan-Verarbeitungsschaltung (siehe [2, 3, 4]) zugeführt werden, worauf im Zusammenhang mit Fig. 4 näher eingegangen wird. Die Ultraschall-Untersuchungsanordnung nach der Erfindung kann also z. B. eine B-Scan-Untersuchungsanordnung enthalten oder als Ergänzung zu ihr vorgesehen sein.

Der Ausgang des Verstärkers 9 ist mit einer Abtastschaltung 10 verbunden, die einen Bandpaß 11 zur Begrenzung der Bandbreite des Meßsignals $\tilde{\Phi}'_s(\vec{r}, t)$ auf etwa die halbe Abtastfrequenz des sich anschließenden, ebenfalls zur Abtastschaltung 10 gehörenden Analog-Digitalwandlers 12 enthält. Dies ist erforderlich, damit die Nyquist-Bedingung eingehalten wird (vergl. z. B. [8], S. 148 ff.).

Der Analog-Digitalwandler 12 tastet dann das in seiner Bandbreite begrenzte Meßsignal $\tilde{\Phi}''_s(\vec{r}, t)$ zur Ermittlung seiner Momentanwerte ab, z. B. mit einer Abtastfrequenz von 2 . . . 5 MHz, und stellt die abgetasteten Momentanwerte digital dar, $\tilde{\Phi}'''_s(\vec{r}, t)$. Bei einer Abtastfrequenz von 2 MHz ergibt sich ein Abtastabstand von 500 ns. Das Auflösungsvermögen des Analog-Digitalwandlers 12 beträgt dabei 12 Bit oder mehr, wobei durch die zuvor erfolgte Verstärkung (Verstärker 9) des Meßsignals gewährleistet ist, daß alle Momentanwerte mit nahezu gleicher relativer Genauigkeit abgetastet werden können.

An den Ausgang des Analog-Digitalwandlers 12 bzw. der Abtastschaltung 10 schließt sich ein Converter 13 an, der eine Formatumwandlung des digitalisierten Meßsignals $\tilde{\Phi}'''_s(\vec{r}, t)$ von der Festkommadarstellung, in der es den Analog-Digitalwandler 12 verläßt, in die Gleitkommadarstellung vornimmt, was ebenfalls pro Abtastpunkt in 500 ns erfolgen kann. Hierdurch wird erreicht, daß nach einem sich an den Converter 13 anschließenden Multiplizierglied 14, in dem das Meßsignal $\tilde{\Phi}'''_s(\vec{r}, t)$ mit dem Kehrwert des Verstärkungsfaktors des Verstärkers 9 multipliziert wird, sämtliche Momentanwerte des so erhaltenen Meßsignals $\tilde{\Phi}''''_s(\vec{r}, t)$ mit der gleichen relativen Genauigkeit digital dargestellt werden können. Der Kehrwert des Verstärkungsfaktors kann z. B. in einem mit dem Multiplizierglied 14 verbundenen Speicher 14a gespeichert sein.

Der Ausgang des Multipliziergliedes 14 ist mit einer Fouriertransformationsschaltung 15 verbunden. Mit ihrer Hilfe wird aus dem Meßsignal $\tilde{\Phi}''''_s(\vec{r}, t)$ ein fouriertransformiertes Meßsignal $\tilde{\Psi}_s(\vec{r}, \omega)$ erhalten. Im Anschluß daran folgt in einem Multiplikationsglied 16, das mit der Fouriertransformationsschaltung 15 verbunden ist, die Multiplikation des fouriertransformierten Meßsignals $\tilde{\Psi}_s(\vec{r}, \omega)$ mit dem Kehrwert des Korrekturfaktors $A(\omega) \cdot B(\omega)$ zur Erzeugung des Frequenzspektrums $\Psi_s(\vec{r}, \omega)$. Der Kehrwert des Korrekturfaktors ist z. B. in dem mit dem Multiplizierglied 16 verbundenen Speicher 16a gespeichert.

Das Frequenzspektrum $\Psi_s(\vec{r}, \omega)$ wird dann zur Ermittlung der inneren Körperstruktur in der elektronischen Einrichtung 21 (Fig. 4) verarbeitet.

Die Fouriertransformation selbst kann ebenfalls in Echtzeit vorgenommen werden. Bei einer angenommenen Eindringtiefe der Schallwellen in den Körper 2 von 20 cm, einer Schallgeschwindigkeit von 1500 m/sec und einem Abtastabstand von 500 ns wird die Signallänge des Meßsignals z. B. zu 512 Abtastpunkte gewählt. Voraussetzung für eine derartige Verarbeitung der Meßsignale ist der Einsatz von sog. »Pipeline FFT-Prozessoren«, die in [5], Kap. 10, genauer beschrieben sind. Bei einer Länge des Meßsignals von 512 Abtastpunkten müssen dazu $\log_2 512 = 9$ »Butterfly-Prozessoren« (vergl. [5], Kap. 10) pro Pipeline FFT-Prozessor hintereinander geschaltet werden. Ihre Taktzeit sollte dabei der Taktzeit der Elemente 12, 13 etc. entsprechen. Für den Fall, daß zwei Meßsignale zu einem »komplexen Signal« zusammengefaßt werden, kann die Hälfte der »Pipeline FFT-Prozessoren« eingespart werden (vergl. [10], Kap. 3), so daß für jeweils zwei Meßstrahlenwege $\overline{M}$ nur eine Fouriertransformationsschaltung 15 erforderlich ist.

In der Fig. 4 ist ein Blockschaltbild der gesamten Ultraschall-Untersuchungsanordnung dargestellt. Die mit den Elementen der Fig. 1 und 3 übereinstimmenden Elemente sind mit jeweils gleichen Bezugsziffern versehen. Mit 3 ist wiederum die Schallwandleranordnung bezeichnet, von der jedoch nur eine Zeile von Schallwandlerelementen 4 dargestellt ist. Die vom Sender 8 erzeugten elektrischen Signale können dabei über einen Verteiler 17 derart verteilt werden, daß alle Schallwandlerelemente 4 gleichzeitig Schallwellen aussenden. Mit Hilfe des Schalters 7 können dann die Schallwandlerelemente 4 ausgewählt werden, deren Meßsignale auf die vorhandenen Meßsignalwege $\overline{M}$ verteilt werden sollen. Schalter 7 bzw. Verteiler 17 können z. B. als integrierte Bausteine ausgebildet sein (vergl. [6]).

In der Fig. 4 sind z. B. vier Meßsignalwege $\overline{M}$ und acht Schallwandlerelemente 4 dargestellt, so daß diese mindestens zweimal zur Aussendung von Schallwellen angeregt werden müssen. In Wirklichkeit kann die Schallwandleranordnung 3 wesentlich mehr Schallwandlerelemente 4 pro Zeile, z. B. 128, besitzen. Die Zeilenzahl kann ebenfalls sehr groß sein, z. B. 16, 32 oder 64 betragen. Es hängt dann von der Zahl der vorhandenen Meßsignalwege $\overline{M}$ ab, wie oft alle Schallwandlerelemente 4 gleichzeitig angeregt werden müssen.

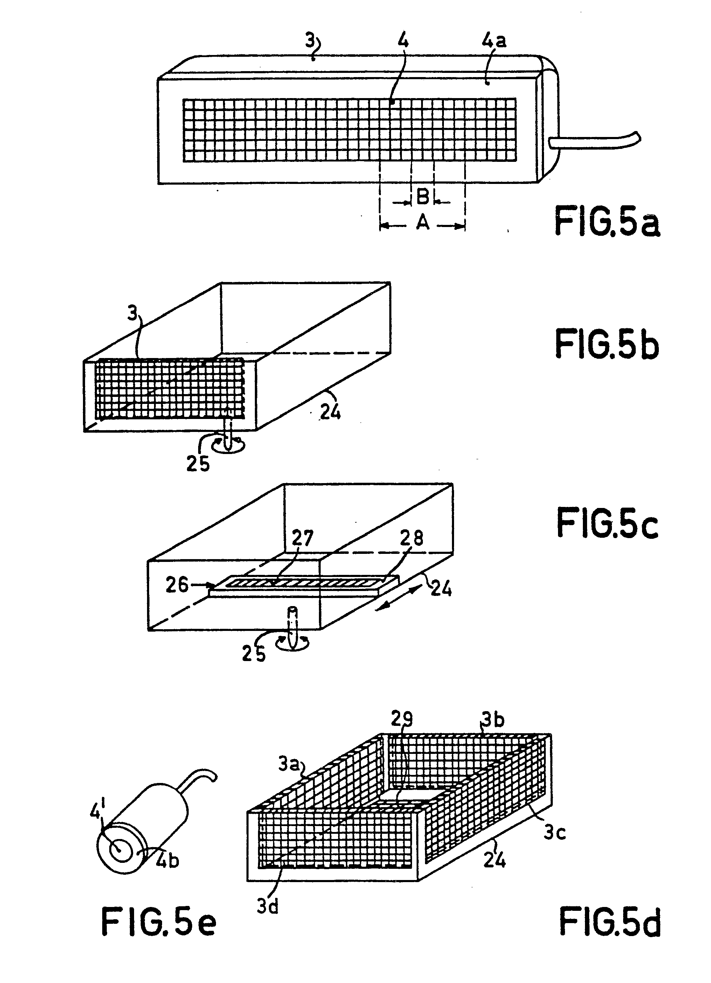

Es ist natürlich auch möglich, daß in Zeilenrichtung fortschreitend jeweils unterschiedliche, zusammenhängende Gruppen A (siehe Fig. 5a) von spaltenförmig angeordneten Schallwandlerelementen 4 zur Aussendung ebener Wellen angeregt werden. Der Schalter 7, der wie der Sender 8 und der Verteiler 17 unter der Kontrolle der Steuereinheit 18 steht, wird dann derart angesteuert, daß zwischen nacheinander erfolgenden Anregungen der Schallwandlerelemente 4 jeweils spaltenweise die im Innern einer Gruppe A liegenden Schallwandlerelemente 4 (Gruppe B) mit den Meßsignalwegen $\overline{M}$ verbunden werden. Für den Fall jedoch, daß die Zahl der Meßsignalwege $\overline{M}$ der Zahl der Schallwandlerelemente 4 entspricht, genügt eine einmalige Aussendung einer Schallwelle, während der Schalter 7 entfallen kann.

Wie schon erwähnt, können die am Ausgang der Verstärker 9 anliegenden Meßsignale $\Phi'_s(r, t)$ zur

6

Ermittlung eines konventionellen B-Scans herangezogen werden. Hierzu sind die Ausgänge der Verstärker 9 mit einer B-Scan-Verarbeitungsschaltung 19 verbunden, die ihrerseits mit der Steuereinheit 18 steuerbar und mit einem Monitor 20 zur Darstellung des B-Scans verbunden ist.

Weiterhin sind die Ausgänge der Multiplikationsglieder 16 mit einer elektronischen Einheit 21 verbunden, die mit Hilfe der Frequenzspektren $\Psi_{s}(\vec{r}, \omega)$ die innere Struktur des Körpers 4 ermittelt, die ebenfalls auf dem Monitor 20 darstellbar ist. Die elektronische Einheit 21 besitzt dabei Massenspeicher 22 zur Aufnahme der pro Meßsignalweg $\overline{M}$ anfallenden Daten sowie eine Recheneinheit 23 zur Berechnung der Körperstruktur aus den Daten nach der oben erwähnten Art. Die Elemente 11 bis 16 werden dabei mittels der Einheit 18 gesteuert. Die Recheneinheit 23 kann z. B. ein konventioneller Mini- oder Mikrocomputer sein, kann aber auch durch einen parallelen Feldrechner, wie er etwa in [7] beschrieben ist, ergänzt werden.

In den Fig. 5a—e sind verschiedene Ausführungsformen von Schallwandleranordnungen für die Ultraschall-Untersuchungsanordnung nach der Erfindung dargestellt. Die in Fig. 5a gezeigte Schallwandleranordnung 3 besitzt Schallwandlerelemente 4, die matrixförmig in einer Ebene angeordnet sind. Sie eignen sich sowohl zum Aussenden als auch zum Empfangen von Schallwellen. Die Schallwandlerelemente 4 besitzen dabei relativ breitbandige Übertragungseigenschaften zur Verarbeitung von impulsförmigen Signalen. Sie sind ferner von einem sog. Guard-Wandler 4a umgeben, der ebenfalls in der Matrixebene liegt und der gleichzeitig mit den Schallwandlerelementen 4 zur Aussendung von Schallwellen angesteuert wird. Durch den Guard-Wandler 4a wird erreicht, daß das durch die Schallwandlerelemente 4 erzeugte Schallwellenfeld besonders eben wird. Die Frontseite der Schallwandleranordnung 3 ist, wie bereits in Fig. 1 dargestellt, mit einer elastischen Folie 6 abgedeckt, zwischen der und der Schallwandleranordnung 3 sich ein Schallkoppelmedium 5, z. B. Wasser, befindet. Natürlich kann der zu untersuchende Körper 4 aber auch über ein Kontaktgel an die Schallwandleranordnung 3 akustisch angekoppelt werden, ohne daß eine derartige Folie erforderlich ist.

Die Schallwandleranordnung 3 kann ferner, wie in Fig. 5b dargestellt, in einem mit einem Schallkoppelmedium füllbaren Tank 24 angeordnet sein und z. B. eine Seitenwand oder den Boden des Tanks 24 darstellen (der Guard-Wandler 4a ist hier der Übersicht wegen nicht eingezeichnet). Der Tank 24 besteht dabei z. B. aus einem rechteckförmigen, wannenartigen Behälter, dessen eine Seite oder dessen Boden von der Schallwandleranordnung 3 gebildet wird, und in den von oben durch eine Öffnung der zu untersuchende Körper einbringbar ist. Der Tank 24 kann dabei um eine Achse 25 drehbar bzw. um den Körper schwenkbar angeordnet sein.

Natürlich kann eine Schallwandleranordnung auch nur aus einer einzigen Zeile 26 von Schallwandlerelementen 27 bestehen, die von einem Guard-Wandler 28 umgeben ist, Fig. 5c. Die Zeile 26 ist dabei auf dem Boden des Tanks 24 angeordnet und senkrecht zur Zeilenrichtung verschiebbar. Ebenso kann eine derartige Zeile auch an einer Seitenwand des Tanks 24 in entsprechender Weise angeordnet sein, z. B. mit ihrer Zeilenrichtung senkrecht zur verlängert gedachten Drehachse 25 liegend.

In dem Tank 24 können sich auch mehrere um die verlängert gedachte Achse 25 herum angeordnete Schallwandleranordnungen befinden. In Fig. 5d sind beispielsweise vier Schallwandleranordnungen 3a—d dargestellt, die die vier Seitenwände des Tanks 24 bilden. Die Schallwandlerelemente 4 einer Schallwandleranordnung 3a können dabei gleichzeitig zur Aussendung einer ebenen Schallwelle angeregt werden, während danach die Schallwandlerelemente 4 aller Schallwandleranordnungen 3a—d zur Ermittlung von Meßsignalen auf Empfang geschaltet werden. Entsprechend können auch vier in einer senkrecht zur Drehachse 25 liegenden Ebene angeordnete Matrixzeilen 26 angesteuert werden.

Nach einer Weiterbildung der Anordnung nach Fig. 5d ist zusätzlich noch der Boden 29 des Tanks 24 mit einer Schallwandleranordnung gemäß Fig. 5b zur Aussendung ebener Schallwellen vollständig bedeckt.

Bei der Untersuchung eines Körpers 4 wird dann nur die auf dem Boden 29 liegende Schallwandleranordnung angeregt, während nach Aussendung der impulsförmigen ebenen Schallwelle alle Schallwandleranordnungen im Tank 24 auf Empfang geschaltet werden. Die auf dem Boden 29 liegende Schallwandleranordnung zum Aussenden ebener Schallwellen kann in diesem Falle aber auch aus einem einzigen plattenförmigen, den Boden vollständig bedeckenden Schallsender bestehen. Zum Empfang der Schallwellen werden dann nur die an den Tankseiten befindlichen Schallwandlerelemente eingeschaltet.

Selbstverständlich kann eine Schallwandleranordnung aber auch aus einem einzigen Schallwandlerelemente 4' bestehen, wie in Fig. 5e dargestellt, das gegenüber den Schallwandlerelementen 4, 27 vergrößert ausgebildet und ggf. von einem Guard-Wandler 4b umgeben ist. Ein derartiges Schallwandlerelement 4' könnte beispielsweise mittels einer mechanischen Führungseinrichtung relativ zum Körper 4 verschiebbar angeordnet sein, z. B. innerhalb des Tanks 24, wobei mit Hilfe der Führungseinrichtung gleichzeitig die jeweiligen Positionen des Schallwandlerelementes 4' zueinander bestimmbar sind. Beispielsweise könnte ein derartiges Element statt einer matrixzeilenförmigen Anordnung verwendet werden, um eine Zeile eines Körpers abzutasten. Zur Darstellung der ermittelten Strukturverteilung des Körpers sind aber die einzelnen Positionen des Schallwandlerelementes entlang der Zeile erforderlich, die dann von der Führungsvorrichtung geliefert werden.

Literatur:

1. US-PS 3 881 466
2. P. N. T. Wells, Biomedical Ultrasonics, Academic Press, London, New York, San Francisco, 1977.
3. J. F. Havlice, J. C. Taenzer, Medical Ultrasonic Imaging: An Overview of Principles and Instrumentation, Proc. IEEE, Vol. 67, 620–641 (1979).
4. R. D. Melen, A. Macovski, J. D. Meindl, Application of Integrated Electronics to Ultrasonic Medical Instruments, Proc. IEEE, Vol. 67, 1274–1285 (1979).
5. L. R. Rabmir, B. Gold, Theory and Application of Digital Signal Processing, Prentice-Hall, Englewood Chiffs, N. J., 1975.
6. J. D. Plummer, J. D. Meindl, A Monolithic 200 — V CMOS Analog Switch, IEEE J. Solid State Circuits, Vol. SC-11, 809–817 (1976).
7. H. Schomberg, A Peripheral Array Computer and its Applications. In: Parallel Computers — Parallel Mathematics (M. Feilmeier, ed.), North-Holland Publishing Company, Amsterdam, 1977.
8. P. Faurre, M. Depeyrot, Elements of Systems Theory, North-Holland Publishing Company, Amsterdam, 1977.
9. R. J. Hanson, J. L. Phillips, Numerical Solution of Two-Dimensional Integral Equations Using Linear Elements, SIAM J. Numer. Anal., Vol. 15, 113–121 (1978).
10. A. v. Oppenheim, R. W. Schaefer, Digital Signal Processing, Prentice-Hall, Englewood Chiffs, N. J., 1975.

## Patentansprüche

1. Anordnung zur Ermittlung der inneren Struktur eines Körpers (2) mit Hilfe von in den Körper eindringenden Ultraschallwellen, mit

a) einer Schallwandleranordnung (3; 3a, 3b, 3c, 3d; 4'; 26), die Schallwellen ($\Phi_i(\dot{r}, t)$) aussendet bzw. empfängt und aus den empfangenen Schallwellen ($\Phi_S(\dot{r}, t)$) Meßsignale ($\tilde{\Phi}_S(\dot{r}, t)$) bildet,

b) einer Steuerschaltung (7), mit der in wenigstens einem aus Senden und Empfangen bestehenden Meßzyklus die Aussendung einer Impulsförmigen Schallwelle vornehmbar ist,

c) einer elektronischen Einrichtung (21) zur Ermittlung der inneren Körperstruktur aus den Meßsignalen, und

d) einer Einheit (19; 20) zur Darstellung der ermittelten Körperstruktur,

e) wobei die Schallwandleranordnung derart ausgebildet bzw. ansteuerbar ist, daß mit ihr pro Meßzyklus eine wenigstens annähernd ebene Schallwelle anregbar ist,

dadurch gekennzeichnet, daß

f) eine elektronische Schaltungseinheit (8) zur Erzeugung impulsförmiger Schallwellen mit breitbandigem Frequenzspektrum vorgesehen ist,

g) daß zusätzlich im Meßsignalweg ($\overline{\text{M}}$) zwischen der Schallwandleranordnung (3; 3a–3d; 4'; 26) und der elektronischen Einrichtung (21) eine Abtastschaltung (10) vorhanden ist, die jedes Meßsignal in sehr kurzen zeitlichen Abständen zur Ermittlung seiner Momentanwerte abtastet und die ermittelten Momentanwerte digitalisiert,

h) Fouriertransformationsschaltungen (15) vorhanden sind, die aus den digitalisierten Momentanwerten jeweils eines Meßsignals ($\tilde{\Phi}_S(\dot{r}, t)$) jeweils ein fouriertransformiertes Meßsignal ($\tilde{\Psi}_S(\dot{r}, \omega)$) erzeugen,

i) zur Ermittlung von Frequenzspektren ($\Psi_S(\dot{r}, \omega)$) Multiplikationsglieder (16) zur Multiplikation der jeweiligen fouriertransformierten Meßsignale ($\tilde{\Psi}_S(\dot{r}, \omega)$) mit dem Kehrwert eines Korrekturfaktors (C) vorgesehen sind, wobei der Korrekturfaktor (C) aus dem Produkt des Frequenzspektrums (A ($\omega$)) der eingestrahlten Schallwelle ($\Phi_i(\dot{r}, t)$) am Ort der Schallsendeelemente und der frequenzabhängigen Übertragungsfunktion (B ($\omega$)) der jeweiligen Empfängerelemente der Ultraschall-Wandlerordnung (3) besteht, zu dem gegebenenfalls ein additiver Wert ($\varepsilon$) addiert wird,

j) und die elektronische Einrichtung (21) derart ausgebildet ist, daß mit ihr wenigstens eine Ermittlung der Verteilung des Brechungsindexes (n ($\dot{r}$)) und/oder des Extinktionskoeffizienten (k ($\dot{r}$)) innerhalb des Körpers mit Hilfe der Frequenzspektren ($\Psi_S(\dot{r}, \omega)$) dadurch erfolgt, daß in der elektronischen Einrichtung (21) eine Verarbeitung der Frequenzspektren ($\Psi_S(\dot{r}, \omega)$) gemäß der Integralgleichung

$$\Psi_S(\dot{r}, \omega) = \int_D G(\dot{r}-\dot{r}', \omega)\, V(\dot{r}', \omega)\, \Psi(\dot{r}', \omega)\, d^3\dot{r}'$$

die die Ausbreitung der am Potential V ($r', \omega$) gestreuten Schallwellen beschreibt, mit

$$\nabla^2 \Psi_s(r, \omega) + \frac{\omega^2}{Co^2} \Psi_s(r, \omega) = V(r, \omega)\, \Psi(r, \omega)$$

$$V(r, \omega) = \omega^2 \cdot f(r) + g(r)$$

und

$$G(r - r', \omega) = -\frac{1}{4\pi}\, \frac{e^{-i\frac{\omega}{Co}|r - r'|}}{|r - r'|}$$

zur Ermittlung der Funktionen $f(r)$ und $g(r)$ vornehmbar ist, die die innere Struktur des Körpers (2) beschreiben, wobei $G(r - r', \omega)$ die Green'sche Funktion zum Differentialoperator

$$\nabla^2 + \frac{\omega^2}{Co^2},\ \Psi(r, \omega)$$

die Summe der Frequenzspektren $(\Psi_i(r, \omega) + \Psi_s(r, \omega))$ von eingestrahlter und gestreuter Schallwelle, $r$ bzw. $r'$ Ortsvektoren, $\omega$ die Frequenz, Co die Geschwindigkeit der Schallwellen in Wasser, D das bestrahlte Gebiet des Körpers (2) und

$$\Psi_i(r, \omega) = e^{i\frac{\omega}{c}x}$$

sind, während

$$f(r) = \frac{1}{Co^2}(1 - \tilde{n}(r)^2)$$

und

$$g(r) = \frac{3}{4}\left(\frac{1}{\rho(r)} \cdot \nabla \rho(r)\right)^2 - \frac{1}{2\,\rho(r)}\nabla^2 \rho(r)$$

ist, wobei

$$\tilde{n}(r) = n(r)(1 + i\,k(r))$$

der komplexe Brechungsindex mit seinem reellen Anteil $n(r)$, $k(r)$ der Extinktionskoeffizient und $\rho(r)$ die Dichte des Körpers ist.

2. Anordnung nach Anspruch 1, dadurch gekennzeichnet, daß die Impulsform der in den Körper (2) eindringenden Ultraschallwellen wenigstens annähernd der einer Deltafunktion entspricht.

3. Anordnung nach Anspruch 1 oder 2, dadurch gekennzeichnet, daß in jedem Meßsignalweg ($\overline{M}$) vor der Abtastschaltung (10) ein Verstärker (9) angeordnet ist, der die Meßsignale ($\Phi_s(r, t)$) proportional zur Laufzeit der Schallwellen im Körper (2) verstärkt, und daß zwischen der Abtastschaltung und der Fouriertransformationsschaltung (15) ein Multiplizierglied (14) angeordnet ist, das das abgetastete und digitalisierte Meßsignal mit dem Kehrwert des Verstärkungsfaktors multipliziert.

4. Anordnung nach einem oder mehreren der Ansprüche 1 bis 3, dadurch gekennzeichnet, daß die Abtastschaltung (10) einen Bandpaß (11) zur Begrenzung der Bandbreite des Meßsignals ($\tilde{\Phi}_s(r, t)$) und eine Analog-Digitalwandler-Einrichtung (12) zum Digitalisieren der Momentanwerte des Meßsignals enthält.

5. Anordnung nach einem oder mehreren der Ansprüche 1 bis 4, dadurch gekennzeichnet, daß in jedem Meßsignalweg ($\overline{M}$) zwischen der Abtastschaltung (10) und dem Multiplizierglied (14) ein Converter (13) zur Formatumwandlung der Meßsignale ($\tilde{\Phi}_s(r, t)$) von der Festkommadarstellung in die Gleitkommadarstellung angeordnet ist.

6. Anordnung nach einem oder mehreren der Ansprüche 1 bis 5, dadurch gekennzeichnet, daß die Schallwandleranordnung aus einem Schallwandlerelement (4') sowohl zur Aussendung als auch zum Empfang von Schallwellen besteht (Fig. 5e).

7. Anordnung nach einem oder mehreren der Ansprüche 1 bis 5, dadurch gekennzeichnet, daß die Schallwandleranordnung (26) aus einer Vielzahl von zeilenförmig angeordneten Schallwandlerelementen (27) zur Aussendung und zum Empfang von Schallwellen besteht (Fig. 5c).

8. Anordnung nach einem oder mehreren der Ansprüche 1 bis 5, dadurch gekennzeichnet, daß die Schallwandleranordnung (3; 3a, 3b, 3c, 3d) aus einer Vielzahl von in Form einer zweidimensionalen Matrix angeordneten Schallwandlerelementen (4) zur Aussendung und zum Empfang von Schallwellen besteht.

9. Anordnung nach einem oder mehreren der Ansprüche 6 bis 8, dadurch gekennzeichnet, daß zur Abtastung eines die Fläche aller Schallwandlerelemente übersteigenden Bereichs des Körpers die Schallwandleranordnung mittels einer mechanischen Führungsvorrichtung verschiebbar angeordnet ist, mit der wenigstens die jeweiligen Positionen der Schallwandleranordnung zueinander bestimmbar sind (Fig. 5c).

10. Anordnung nach einem oder mehreren der Ansprüche 6 bis 8, dadurch gekennzeichnet, daß die dem Körper (2) zugewandte Seite der Schallwandleranordnung (3) mit einer elastischen Folie (6) bedeckt ist, und daß sich zwischen der Folie und der Schallwandleranordnung ein Schallkoppelmedium (5) befindet (Fig. 1).

11. Anordnung nach Anspruch 6, 7 oder 8, dadurch gekennzeichnet, daß die Schallwandleranordnung (3, 26) im Innern eines mit einem Schallkoppelmedium füllbaren Tanks (24) zur Aufnahme des zu untersuchenden Körpers (2) angeordnet ist (Fig. 5b–5d).

12. Anordnung nach Anspruch 11, dadurch gekennzeichnet, daß der Tank (24) relativ zum Körper (2) drehbar und/oder schwenkbar angeordnet ist und daß die verlängert gedachte Drehachse (25) die Tanköffnung zur Aufnahme des Körpers durchsetzt.

13. Anordnung nach einem der Ansprüche 6 bis 8, und 11, 12, dadurch gekennzeichnet, daß sich im Innern des Tanks (24) mehrere Schallwandleranordnungen (3a–d, 26) befinden, die um die verlängert gedachte Drehachse (25) herum angeordnet sind.

14. Anordnung nach Anspruch 13, dadurch gekennzeichnet, daß die Schallwandleranordnungen (3a–d, 26) einander entsprechen und jeweils gleiche Zeilen von Schallwandlerelementen in gleichen, senkrecht zur Drehachse (25) liegenden Ebenen angeordnet sind.

15. Anordnung nach Anspruch 7 bzw. 8 und 13, dadurch gekennzeichnet, daß zusätzlich eine Schallwandleranordnung auf dem Boden (29) des Tanks (24) angeordnet ist.

16. Anordnung nach Anspruch 7 und 15, dadurch gekennzeichnet, daß die auf dem Boden liegende Schallwandleranordnung senkrecht zur ihrer Zeilenrichtung verschiebbar angeordnet ist.

17. Anordnung nach Anspruch 15, dadurch gekennzeichnet, daß die auf dem Boden (29) liegende Schallwandleranordnung zum Aussenden von ebenen Schallwellen aus einem einzigen plattenförmigen Schallwandlerelement besteht.

18. Anordnung nach einem oder mehreren der Ansprüche 7 bis 11, dadurch gekennzeichnet, daß alle Schallwandlerelemente (4) zur Aussendung ebener Schallwellen gleichzeitig periodisch anregbar sind, und daß eine Auswahlschaltung (7) vorgesehen ist, mit der zwischen nacheinander erfolgenden Anregungen jeweils unterschiedliche Gruppen von Schallwandlerelementen zum Empfang von Schallwellen einschaltbar sind (Fig. 4).

19. Anordnung nach einem oder mehreren der Ansprüche 7 bis 11, dadurch gekennzeichnet, daß in Zeilenrichtung fortschreitend jeweils unterschiedliche, zusammenhängende Gruppen von spaltenförmig angeordneten Schallwandlerelementen (4) zur Aussendung ebener Schallwellen anregbar sind, und daß eine Auswahlschaltung (7) vorgesehen ist, mit der zwischen nacheinander erfolgenden Anregungen jeweils spaltenweise die im Innern einer Gruppe liegenden Schallwandlerelemente zum Empfang von Schallwellen einschaltbar sind.

20. Anordnung nach einem oder mehreren der Ansprüche 13 bis 15, dadurch gekennzeichnet, daß pro Meßzyklus alle Schallwandlerelemente (4) nur einer Schallwandleranordnung zur Aussendung einer ebenen Schallwelle gleichzeitig anregbar sind, und daß anschließend die Schallwandlerelemente aller Schallwandleranordnungen (3a, 3b, 3c, 3d) zum Empfang von Schallwellen einschaltbar sind (Fig. 5d).

21. Anordnung nach einem der vorhergehenden Ansprüche, dadurch gekennzeichnet, daß die Zahl der zum Empfang von Schallwellen einschaltbaren Schallwandlerelemente (4) der Zahl der Meßsignalwege ($\overline{M}$) entspricht.

22. Anordnung nach einem der vorhergehenden Ansprüche, dadurch gekennzeichnet, daß für jeweils zwei Meßsignalwege ($\overline{M}$) eine Fouriertransformationsschaltung (15) vorgesehen ist.

23. Anordnung nach einem der vorhergehenden Ansprüche, dadurch gekennzeichnet, daß die zum Aussenden von Schallwellen vorgesehene Schallwandleranordnung (3; 4'; 26) von einem Guard-Wandler (4a; 4b; 28) umgeben ist.

## Claims

1. A device for determining the internal structure of a body (2) by means of ultrasonic waves which penetrate the body, comprising

a) an acoustic transducer device (3; 3a, 3b, 3c, 3d; 4', 26) which emits and receives acoustic waves ($\Phi_i(\bar{r}, t)$) and forms measurement signals ($\Phi_S(\bar{r}, t)$) from the acoustic waves ($\Phi_S(\bar{r}, t)$) received,

b) a control circuit (17) whereby the emission of a pulse-shaped acoustic wave can be performed in at least one measurement cycle consisting of emission and reception,

c) an electronic device (21) for determining the internal body structure from the measurement signals, and

d) a unit (19, 20) for the display of the body structure determined,

e) the acoustic transducer device being constructed and activatable so that for each measurement cycle an at least approximately planar acoustic wave can be excited thereby,

characterized in that

f) an electronic circuit (8) is provided for generating pulse-shaped acoustic waves having a wide-band frequency spectrum,

g) in the measurement signal path ($\overline{M}$) between the acoustic transducer device (3; 3a–3d; 4', 26) and the electronic device (21) there is provided a sampling circuit (10) which samples each measurement signal at very short time intervals in order to determine the instantaneous values thereof and which digitizes the instantaneous values determined,

h) there are provided Fourier transformation circuits (15) which form a respective Fourier transformed measurement signal ($\tilde{\Psi}_s(\vec{r}, \omega)$) from the digitized instantaneous values each time a measurement signal ($\tilde{\Phi}_s(\vec{r}, t)$),

i) for the determination of frequency spectra ($\tilde{\Psi}_s(\vec{r}, \omega)$) there are provided multiplier elements (16) for multiplying the relevant Fourier transformed measurement signals ($\tilde{\Psi}_s(\vec{r}, \omega)$) by the reciprocal value of a correction factor (C), the correction factor (C) consisting of the product of the frequency spectrum (A($\omega$)) of the emitted acoustic wave ($\Phi_1(\vec{r}, t)$) at the location of the acoustic emission elements and the frequency-dependent transmission function (B($\omega$)) of the relevant receiver elements of the ultrasonic transducer device (3), whereto possibly an additive value ($\varepsilon$) is added,

j) and the electronic device (21) is constructed so that thereby at least the distribution of the refractive index (n($\vec{r}$)) and/or the extinction coefficient (k($\vec{r}$)) within the body can be obtained by means of the frequency spectra (($\Psi_s(\vec{r}, \omega)$), in that in the electronic device (21) the frequency spectra ($\Psi_s(\vec{r}, \omega)$) are processed in accordance with the integral equation

$$\Psi_s(\vec{r}, \omega) = \int_D G(\vec{r}-\vec{r}', \omega)\, V(\vec{r}', \omega)\, \Psi(\vec{r}', \omega)\, d^3\vec{r}'$$

which describes the propagation of the acoustic waves scattered by the potential V($\vec{r}'$, $\omega$), with

$$\nabla^2\, \Psi_s(\vec{r}, \omega) + \frac{\omega^2}{Co^2}\, \Psi_s(\vec{r}, \omega) = V(\vec{r}, \omega)\, \Psi(\vec{r}, \omega)$$

$$V(\vec{r}, \omega) = \omega^2 \cdot f(\vec{r}) + g(\vec{r})$$

and

$$G(\vec{r} - \vec{r}', \omega) = -\frac{1}{4\pi}\, \frac{e^{-i\frac{\omega}{Co}\,\vec{r}\cdot\vec{r}'}}{|\vec{r} - \vec{r}'|}$$

in order to determine the functions f(r) and g($\vec{r}$) which describe the internal structure of the body (2), G($\vec{r} - \vec{r}'$, $\omega$) being Green's function for the differential operator

$$\nabla^2 + \frac{\omega^2}{Co^2}, \ \Psi(\vec{r}, \omega)$$

being the sum of the frequency spectra ($\Psi_1(\vec{r}, \omega) + \Psi_s(\vec{r}, \omega)$) of the emitted and scattered acoustic wave, $\vec{r}$ and r' being location vectors, $\omega$ being the frequency, Co the velocity of the acoustic waves in water, D being the scanned part of the body (2) and

$$\Psi_1(\vec{r}, \omega) = e^{i\frac{\omega}{c}x}$$

whilst

$$f(\vec{r}) = \frac{1}{Co^2}\, (1 - \tilde{n}(\vec{r})^2)$$

and

$$g(\vec{r}) = \frac{3}{4}\, \left(\frac{1}{G(\vec{r})} \cdot \nabla G(\vec{r})\right)^2 - \frac{1}{2\, G(\vec{r})}\, \nabla^2 G(\vec{r})$$

where

$$\tilde{n}(\vec{r}) = n(\vec{r})\, (1 + i\, k(\vec{r}))$$

is the complex refractive index with its real part n (r̃), k (r̃) being the extinction coefficient, and G (r̃) being the density of the body.

2. A device as claimed in Claim 1, characterized in that the pulse shape of the ultrasonic waves penetrating the body (2) corresponds at least approximately to a delta function.

3. A device as claimed in Claim 1 or 2, characterized in that the sampling circuit (10) in each measurement signal path ($\overline{M}$) is preceded by an amplifier (9) which amplifies the measurement signals ($\tilde{\Phi}_S$ (r̃, t)) in proportion to the delay time of the acoustic waves in the body (2) and a multiplied element (14) is arranged between the sampling circuit and the Fourier transformation circuit (15), which element multiplies the sampled and digitized measurement signal by the reciprocal value of the gain factor.

4. A device as claimed in one or more of the Claims 1 to 3, characterized in that the sampling circuit (10) comprises a bandpass filter (11) for limiting the bandwidth of the measurement signal ($\tilde{\Phi}_S$ (r̃, t)) and an analog-to-digital converter (12) for digitizing the instantaneous values of the measurement signal.

5. A device as claimed in one or more of the Claims 1 to 4, characterized in that between the sampling circuit (10) and the multiplier element (14) in each measurement signal path ($\overline{M}$) there is provided a converter (13) for converting the format of the measurement signals ($\tilde{\Phi}_S$ (r̃, t)) from the fixed-point representation to the floating-point representation.

6. A device as claimed in one or more of the Claims 1 to 5, characterized in that the acoustic transducer device consists of a single acoustic transducer element (4') for the emission as well as for the reception of acoustic waves (figure 5e).

7. A device as claimed in one or more of the Claims 1 to 5, characterized in that the acoustic transducer device (26) consists of a plurality of row-wise arranged acoustic transducer elements (27) for the emission and the reception of acoustic waves (figures 5c).

8. A device as claimed in one or more of the Claims 1 to 5, characterized in that the acoustic transducer device (3; 3a, 3b, 3c, 3d) consists of a plurality of acoustic transducer elements (4) for the emission and the reception of acoustic waves, which are arranged in the form of a two-dimensional matrix.

9. A device as claimed in one or more of the Claims 1 to 8, characterized in that for scanning a part of the body which is larger than the surface aera of all the acoustic transducer elements, the acoustic transducer device is arranged to be displaceable be means of a mechanical guide device whereby at least the relevant positions of the acoustic transducer device with respect to each other can be determined (figure 5c).

10. A device as claimed in one or more of the Claims 6 to 8, characterized in that the side of the acoustic transducer device (3) which faces the body (2) is covered with an elastic foil (6), an acoustic coupling medium (5) being present between the foil and the acoustic transducer device (figure 1).

11. A device as claimed in Claim 6, 7 or 8, characterized in that the acoustic transducer device (3, 26) is arranged inside a tank (24) which is to be filled with an acoustic coupling medium and which serves to accomodate the body (2) to be examined (figures 5b–5d).

12. A device as claimed in Claim 11, characterized in that the tank (24) is arranged to be rotatable and/or pivotable with respect to the body (2), the notional extension of the rotary shaft (25) extending through the tank opening for accomodating the body.

13. A device as claimed in any one of the Claims 6 to 8 and 11, 12, characterized in that inside the tank (24) there are arranged several acoustic transducer devices (3a–d, 26) which are arranged about the notional extension of the rotary shaft (25).

14. A device as claimed in Claim 13, characterized in that the acoustic transducer devices (3a–d, 26) correspond with one another and in each case the same rows of acoustic transducer elements are arranged in the same planes which extend perpendicularly to the rotary shaft (25).

15. A device as claimed in Claim 7 or 8 and 13, characterized in that an additional acoustic transducer device is arranged on the bottom (29) of the tank (24).

16. A device as claimed in Claims 7 and 15, characterized in that the acoustic transducer device arranged on the bottom is displaceable perpendicularly to its row direction.

17. A device as claimed in Claim 15, characterized in that the acoustic transducer device arranged on the bottom (29) consists of a single, plate-shaped acoustic transducer element for the emission of planar acoustic waves.

18. A device as claimed in one or more of the Claims 7 to 11, characterized in that all acoustic transducer elements (4) for the emission of planar acoustic waves can be simultaneously, periodically activated, a selection circuit (7) being provided whereby respective different groups of acoustic transducer elements can be activated for the reception of acoustic waves between successively performed activations (figure 4).

19. A device as claimed in one or more of the Claims 7 to 11, characterized in that, proceding in the row direction respective different associated groups of column-wise arranged acoustic transducer elements (4) can be activated for the emission of planar acoustic waves, a selection circuit (7) being provided whereby each time, between successively executed activations, acoustic transducer elements situated within a group can be column-wise activated for the reception of acoustic waves.

20. A device as claimed in one or more of the Claims 13 to 15, characterized in that for each measurement cycle all the acoustic transducer elements (4) of only one acoustic transducer device can be simultaneously activated for the emission of a planar acoustic wave, whilst subsequently the acoustic transducer elements of all the acoustic transducer devices (3a, 3b, 3c, 3d) can be activated for the reception of acoustic waves (figure 5d).

21. A device as claimed in any one of the preceding Claims, characterized in that the number of acoustic transducer elements (4) which can be switched on for the reception of acoustic waves corresponds to the number of measurement signal paths ($\overline{M}$).

22. A device as claimed in any one of the preceding Claims, characterized in that a Fourier transformation circuit (15) is provided for each pair of measurement signal paths ($\overline{M}$).

23. A device as claimed in any one of the preceding Claims, characterized in that the acoustic transducer device (3; 4', 26) for the emission of acoustic waves is surrounded by a guard transducer (4a; 4b; 28).

## Revendications

1. Dispositif de détermination d'une structure interne d'un corps (2) au moyen d'ondes ultrasonores pénétrant dans le corps, comportant

a) un dispositif transducteur acoustique (3; 3a; 3b; 3c; 3d; 4'; 26) émettant et recevant les ondes acoustiques ($\Phi_i(\dot{r}, t)$) et le formant des signaux de mesure ($\Phi_{s}(\dot{r}, t)$) à partir des ondes acoustiques reçues ($\tilde{\Phi}_{s}(\dot{r}, t)$),

b) un circuit de commande (7) permettant d'effectuer l'émission d'une onde acoustique impulsionnelle dans au moins un cycle de mesure consistant et une émission et en une réception,

c) un dispositif électronique (21) de détermination de la structure interne du corps à partir des signaux de mesure et

d) une unité (19, 20) d'affichage de la structure déterminée du corps,

e) (le dispositif transducteur électro-acoustique étant réalisé et pouvant être commandé de façon à permettre, au cours de chaque cycle de mesure, d'émettre une onde acoustique au moins pratiquement plane,
caractérisé

f) en ce qu'il est prévu un circuit électronique (8) pour la génération d'ondes acoustiques impulsionnelles ayant un spectre de fréquence à large bande,

g) en ce que dans le trajet de signal de mesure ($\overline{M}$) entre le dispositif transducteur (3; 3a–3d; 4'; 26) et le dispositif électronique (21), est prévu un circuit d'échantillonnage (10) qui échantillonne chaque signal de mesure à des intervalles de temps très courts pour déterminer ses valeurs momentanées et qui numérise les valeurs momentanées déterminées.

h) en ce qu'il est prévu des circuits de transformation de Fourier (15) qui, à partir des valeurs momentanées numérisées de chaque signal de mesure ($\tilde{\Phi}_{s}(\dot{r}, t)$), forme un signal de mesure à transformation de Fourier ($\tilde{\Psi}_{s}(\dot{r}, \omega)$),

i) en ce que, pour la détermination de spectres de fréquence ($\Psi_{s}(\dot{r}, \omega)$), sont prévus des éléments multiplicateurs (16) servant à multiplier chaque signal de mesure à transformation de Fourier ($\tilde{\Psi}_{s}(\dot{r}, \omega)$) par la valeur inverse d'un facteur de correction (C), le facteur de correction (C) étant constitué par le produit du spectre de fréquence $A(\omega)$ de l'onde acoustique ($\Phi_i(\dot{r}, t)$) émise à l'endroit des éléments émetteurs acoustiques et de la fonction de transmission dépendante de la fréquence ($B(\omega)$) des éléments récepteurs concernés du dispositif transducteur ultrasonore (3), fonction à laquelle on ajoute, le cas échéant, une valeur additive ($\varepsilon$).

j) et en ce que le dispositif électronique (21) est réalisé de façon à effectuer au moins une détermination de la répartition de l'indice de réfraction (n) ($\dot{r}$) et/ou du coefficient d'extinction (k ($\dot{r}$)) dans le corps à l'aide des spectres de fréquence ($\Psi_{s}(\dot{r}, \omega)$) du fait que, dans le dispositif électronique (21), peut être réalisé un traitement des spectres de fréquence ($\Psi_{s}(\dot{r}, \omega)$) selon l'équation intégrale

$$\Psi_{s}(\dot{r}, \omega) = \int_{D} G(\dot{r}-\dot{r}'', \omega)\, V(\dot{r}, \omega)\, \Psi(\dot{r}', \omega)\, d^3\dot{r}'$$

qui décrit la propagation des ondes acoustiques diffusées au potentiel ($V(\dot{r}', \omega)$ avec

$$\nabla^2\, \Psi_{s}(\dot{r}, \omega) + \frac{\omega^2}{Co^2}\, \Psi_{s}(\dot{r}, \omega) = V(\dot{r}, \omega)\, \Psi(\dot{r}, \omega)$$

$$V(\dot{r}, \omega) = \omega^2 \cdot f(\dot{r}) + g(\dot{r})$$

et

13

$$G(\vec{r} - \vec{r}', \omega) = -\frac{1}{4\pi} \frac{e^{-i\frac{\omega}{Co}|\vec{r}-\vec{r}'|}}{|\vec{r}-\vec{r}'|}$$

pour déterminer les fonctions $f(\vec{r})$ et $g(\vec{r})$ décrivant la structure interne du corps (2), $G(\vec{r}-\vec{r}', \omega)$ étant la fonction de Green pour l'opérateur différéntiel

$$\nabla^2 + \frac{\omega^2}{Co^2}, \ \Psi(\vec{r}, \omega)$$

étant la somme des spectres de fréquence ($\Psi_i(\vec{r}, \omega) + \Psi_s(\vec{r}, \omega)$) d'ondes acoustiques émises et diffusées, $\vec{r}$ et $\vec{r}'$ étant des rayons vecteurs, $\omega$ étant la fréquence, Co étant la vitesse des ondes acoustiques dans de l'eau, D étant la zone explorée du corps (2) et

$$\Psi_i(\vec{r}, \omega) = e^{i\frac{\omega}{c}x}$$

alors que

$$f(\vec{r}) = \frac{1}{Co^2}(1 - \tilde{n}(\vec{r})^2)$$

et

$$g(\vec{r}) = \frac{3}{4}\left(\frac{1}{\rho(\vec{r})} \cdot \nabla \rho(\vec{r})\right)^2 - \frac{1}{2\,\rho(\vec{r})}\nabla^2\,\rho(\vec{r})$$

et que

$$\tilde{n}(\vec{r}) = n(\vec{r})(1 + i\,k(\vec{r}))$$

est l'indice de réfraction complexe avec sa part réelle $n(\vec{r})$, $k(\vec{r})$ est le coefficient d'extinction et $\rho(\vec{r})$ est la densité du corps.

2. Dispositif selon la revendication 1, caractérisé en ce que la forme impulsionnelle des ondes ultrasonores pénétrant dans le corps (2) correspond au moins pratiquement à une fonction delta.

3. Dispositif selon la revendication 1 ou 2, caractérisé en ce que dans chaque trajet de signal de mesure ($\overline{M}$), le circuit d'échantillonnage (10) est précédé d'un amplificateur (9) qui amplifie les signaux de mesure ($\tilde{\Phi}_s(\vec{r}, t)$) en proportion du temps de propagation des ondes acoustiques dans le corps (2) et en ce qu'entre le circuit d'échantillonnage et le circuit de transformation de Fourier (15), est disposé un élément multiplicateur (14) qui multiplie le signal de mesure échantillonné et numérisé par l'inverse du taux d'amplification.

4. Dispositif selon une ou plusieurs des revendications 1 à 3, caractérisé en ce que le circuit d'échantillonnage (10) comporte, d'une part, un filtre passe-bande (11) pour limiter la largeur de bande du signal de mesure ($\tilde{\Phi}_s(\vec{r}, t)$) et, d'autre part, un convertisseur analogique-numérique (12) pour numériser les valeurs momentanées du signal de mesure.

5. Dispositif selon un ou plusieurs des revendications 1 à 4, caractérisé en ce que, dans chaque trajet de signal de mesure ($\overline{M}$), entre le circuit d'échantillonnage (10) et l'élément multiplicateur (14), est disposé un convertisseur (13) servant à convertir le format des signaux de mesure ($\tilde{\Phi}_s(\vec{r}, t)$) à partir de la représentation en virgule fixe en la représentation en virgule flottante.

6. Dispositif selon une ou plusieurs des revendications 1 à 5, caractérisé en ce que le dispositif transducteur acoustique est constitué par un seul élément transducteur acoustique (4') servant à émettre et à recevoir des ondes acoustiques (figure 5e).

7. Dispositif selon une ou plusieurs des revendications 1 à 5, caractérisé en ce que le dispositif transducteur acoustique (26) est constitué par plusieurs éléments transducteurs acoustiques (27) disposés suivant un alignement et servant à émettre et à recevoir des ondes acoustiques (figure 5c).

8. Dispositif selon une ou plusieurs des revendications 1 à 5, caractérisé en ce que le dispositif transducteur acoustique (3; 3a, 3b, 3c, 3d) est constitué par plusieurs éléments transducteurs acoustiques (4) disposés suivant une matrice bidimensionnelle et servant à émettre et à recevoir des ondes acoustiques.

Dispositif selon une ou plusieurs des revendications 6 à 8, caractérisé en ce que pour l'exploration d'une zone de corps d'une étendue plus grande que la surface de tous les éléments transducteurs acoustiques, le dispositif transducteur acoustique peut être déplacé au moyen d'un dispositif mécanique de guidage permettant de déterminer au moins les différentes positions du dispositif transducteur acoustique les unes par rapport aux autres (figure 5c).

10. Dispositif selon une ou plusieurs des revendications 6 à 8, caractérisé en ce que la face du dispositif transducteur acoustique (3) située en regard du corps (2) est recouverte d'une feuille élastique (6) et en ce qu'un milieu de couplage acoustique (5) est situé entre la feuille et le dispositif transducteur acoustique (figure 1).

11. Dispositif selon la revendication 6, 7 ou 8, caractérisé en ce que le dispositif transducteur acoustique (3a–d, 26) est disposé dans un réservoir (24) pouvant être rempli d'un milieu de couplage acoustique et servant à recevoir le corps à examiner (2) (figures 5b à 5d).

12. Dispositif selon la revendication 11, caractérisé en ce que le réservoir (24) peut tourner et/ou pivoter par rapport au corps (2) et en ce que le prolongement imaginaire de l'axe de rotation (25) traverse l'ouverture du réservoir pour la réception du corps.

13. Dispositif selon l'une des revendications 6 à 8 et 11, 12, caractérisé en ce que dans le réservoir (24), sont situés plusieurs dispositifs transducteurs acoustiques (3a–d, 26) disposés autour du prolongement imaginaire de l'axe de rotation (25).

14. Dispositif selon la revendication 13, caractérisé en ce que les dispositifs transducteurs acoustiques (3a–3d, 26) correspondent les uns aux autres de façon que, dans les différents dispositifs les mêmes rangées d'éléments transducteurs acoustiques soient disposées dans les mêmes plans, perpendiculaires à l'axe de rotation (25).

15. Dispositif selon les revendications 7 ou 8 et 13, caractérisé en ce qu'un dispositif transducteur acoustique additionnel est disposé sur le fond (29) du réservoir (24).

Dispositif selon les revendication 7 et 15, caractérisé en ce que le dispositif transducteur acoustique disposé sur le fond peut être déplacé perpendiculairement à son sens d'alignement.

17. Dispositif selon la revendication 15, caractérisé en ce que le dispositif transducteur acoustique situé sur le fond (29) est constitué par un seul élément transducteur acoustique en forme de plaque pour l'émission d'ondes acoustiques planes.

18. Dispositif selon une ou plusieurs des revendications 7 à 11, caractérisé en ce que tous les éléments transducteurs acoustiques (4) peuvent être excités simultanément et périodiquement et en ce qu'il est prévu un sélecteur (7) permettant, entre des excitations successives, d'actionner chaque fois des groupes différents d'éléments transducteurs acoustiques pour la réception d'ondes acoustiques (figure 4).

19. Dispositif selon une ou plusieurs des revendications 7 à 11, caractérisé en ce que, des groupes distincts associés d'éléments transducteurs acoustiques (4) disposés en colonnes peuvent être excités successivement dans le sens des lignes pour l'émission d'ondes acoustiques planes et en ce qu'il est prévu un sélecteur (7) par lequel, entre des excitations successives, les éléments transducteurs acoustiques situés dans un groupe peuvent être actionnés colonne par colonne pour la réception d'ondes acoustique.

20. Dispositif selon une ou plusieurs des revendications 13 à 15, caractérisé en ce que, dans chaque cycle de mesure, tous les éléments transducteur acoustiques (4) d'un seul dispositif transducteur acoustique peuvent être excités simultanément pour l'émission d'une onde acoustique plane et en ce qu'ensuite, les éléments transducteurs acoustiques de tous les dispositifs transducteurs acoustiques (3a, 3b, 3c; 3d) peuvent être enclenchés pour la réception d'ondes acoustiques (figure 5d).

21. Dispositif selon l'une quelconque des revendications précédentes, caractérisé en ce que le nombre d'éléments transducteurs acoustiques (4) pouvant être enclenchés pour la réception d'ondes acoustiques correspond au nombre des trajets de signal de mesure (M̄).

22. Dispositif selon l'une quelconque des revendications précédentes, caractérisé en ce qu'un circuit de transformation de Fourier (15) est prévu pour chaque paire de trajets de signal de mesure (M̄).

23. Dispositif selon l'une quelconque des revendications précédentes, caractérisé en ce que le dispositif transducteur acoustique (3; 4', 26) prévu pour l'émission d'ondes acoustiques est entouré d'un transducteur de garde (4a; 4b; 28).

15

**FIG.1**

**FIG.2**

FIG.3

$$\psi_S(\vec{r},\omega)= \frac{\widetilde{\psi}_S(\vec{r},\omega)}{A(\omega)\,B(\omega)}$$

FIG.4

FIG.5a

FIG.5b

FIG.5c

FIG.5e

FIG.5d